# FASCICULE DE BREVET EUROPEEN

(11) **EP 2 476 399 B1**
(45) Date de publication et mention de la délivrance du brevet: **21.08.2019**
(21) Numéro de dépôt: 11290571.6
(22) Date de dépôt: 12.12.2011
(51) Int. Cl.: A61F 9/00, A61F 9/007

(54) **Canule autobloquante**
Selbstblockierende Kanüle
Self-blocking nozzle

(30) Priorité: 18.01.2011 FR 1100141
(43) Date de publication de la demande: 18.07.2012
(73) Titulaire: France Chirurgie Instrumentation SAS-FCI, 75008 Paris (FR)
(72) Inventeur: Zech, Jean-Christophe, 69009 Lyon (FR)
(74) Mandataire: Eidelsberg, Olivier Nathan

(56) Documents cités:
- EP-A2- 1 996 092
- WO-A1-96/36377
- WO-A1-2010/126076
- FR-A1- 2 830 186
- JP-A- 2007 319 423
- US-A- 5 300 020
- US-A1- 2008 177 239

## Description

La présente invention se rapporte à un ensemble constitué d'au moins une canule destinée à traverser la conjonctive et/ou la sclère de l'oeil pour permettre le passage d'un outil destiné à avoir une action dans le volume intraoculaire, tel qu'un instrument d'aspiration, une ligne d'infusion, un instrument de coupe, par exemple à grande vitesse, du gel vitreux contenu dans le volume intraoculaire, un guide lumineux destiné à transmettre de la lumière pour voir ce qui se passe dans le volume intraoculaire ou analogue, et d'un outil du genre mentionné précédemment ayant les dimensions appropriées pour pouvoir passer par la canule.

Les ensembles de ce genre de l'art antérieur ne sont pas simples à utiliser par le chirurgien. En particulier, le mouvement du chirurgien pour déplacer les outils manque souvent de précision et par exemple lorsque le chirurgien fait passer une ligne d'infusion par la canule, il peut arriver, en raison des manipulations de l'oeil, que l'ensemble canule et ligne ressorte vers l'extérieur et du liquide physiologique en vient à être injecté sous la rétine, ce qui n'est pas souhaitable et peut provoquer un décollement de rétine.

La présente invention vise à surmonter les inconvénients de l'art antérieur en proposant un ensemble du genre mentionné ci-dessus, qui donne au chirurgien une plus grande sécurité lorsqu'il manoeuvre son outil dans la cavité intraoculaire, ce qui a pour effet d'améliorer la sécurité pour le patient et d'accélérer les opérations effectuées.

Suivant l'invention, une canule est telle que définie à la revendication 1, des perfectionnements étant définis aux sous-revendications 2 à 5.

En prévoyant ainsi de bloquer en position la canule lorsqu'elle est positionnée à travers la sclère et/ou la conjonctive pour former un passage à travers ces parois pour l'outil, on s'assure que la canule reste bien stable en position, ce qui permet de lutter contre une sortie intempestive de la canule et de l'outil qui la traverse, notamment une ligne de perfusion, pendant les manipulation de l'outil ou de l'oeil, ce qui permet d'éviter les conséquences néfastes liées à une telle sortie intempestive, par exemple une injection de liquide sous la rétine dans le cas où l'outil associé est une ligne de perfusion. La canule est ainsi autobloquante.

Suivant un mode de réalisation préféré de l'invention et notamment particulièrement simple, il est prévu des moyens de blocage sous la forme d'une gorge annulaire formée à l'extérieur de la canule.

Suivant un mode de réalisation préféré de l'invention, la gorge est formée par deux butées annulaires faisant saillie latéralement de la canule à distance l'une de l'autre, de manière à former entre elles la gorge annulaire.

Suivant un mode de réalisation préféré de l'invention, l'extrémité distale de la canule, c'est-à-dire l'extrémité par laquelle pénètre la canule dans l'espace intraoculaire, a une forme biseautée.

Ce biseau favorise une mise en place progressive et non traumatique de la canule à travers la sclère.

Une canule selon le préambule de la revendication 1 est connue du document US-A-2008/0177239.

La présente invention se rapporte également à un ensemble constitué d'au moins une canule suivant l'invention et d'au moins un outil destiné à avoir une action dans le volume intraoculaire, tel qu'un instrument d'aspiration, un instrument de coupe, par exemple à grande vitesse, du gel vitreux contenu dans le volume intraoculaire, un guide lumineux destiné à transmettre de la lumière pour voir ce qui se passe dans le volume intraoculaire ou analogue, et ayant les dimensions appropriées pour pouvoir passer dans le canal de la canule ainsi qu'à un ensemble constitué d'au moins une canule suivant l'invention et d'au moins un outil destiné à avoir une action dans le volume intraoculaire, tel qu'un instrument d'aspiration, un instrument de coupe, par exemple à grande vitesse, du gel vitreux contenu dans le volume intraoculaire, un guide lumineux destiné à transmettre de la lumière pour voir ce qui se passe dans le volume intraoculaire ou analogue, et passant dans le canal de la canule.

A titre d'exemple, on décrit maintenant des modes de réalisation préférés de l'invention en se reportant aux dessins, dans lesquels :
- la Figure 1: est une vue en coupe schématique d'un oeil, dans lequel sont introduits deux outils par l'intermédiaire de deux canules suivant l'invention ;
- la Figure 2: est une vue agrandie d'une partie de la Figure 1 ;
- la Figure 3: est une vue en perspective de la canule des figures 1 et 2;
- la Figure 4: est une vue en perspective d'un autre mode de réalisation d'une canule suivant l'invention ; et
- la Figure 5: est une vue en perspective d'encore un autre mode de réalisation d'une canule suivant l'invention.

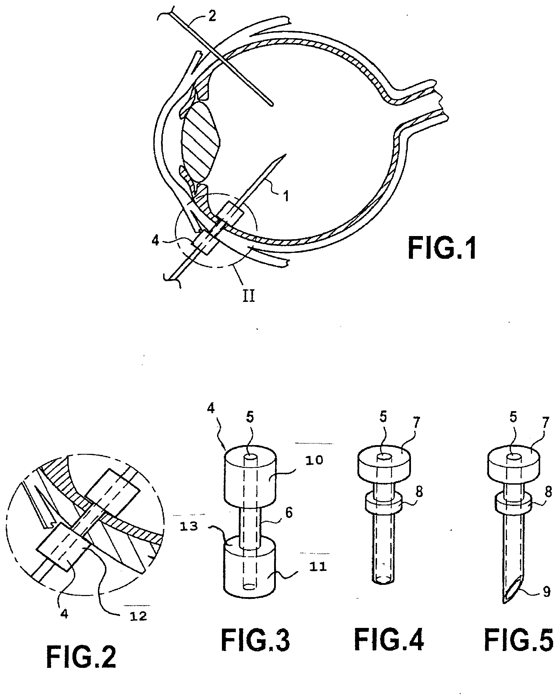

Les figures 1 à 3 ne représentent pas un mode de réalisation de l'invention.

A la Figure 1, il est représenté schématiquement une vue en coupe d'un oeil. Des outils 1 et 2, respectivement un outil 1 d'aspiration et un outil 2 formant guide de lumière pour permettre au chirurgien de voir ce qu'il fait avec l'outil 1, sont insérés dans l'espace intraoculaire de l'oeil. Chaque outil 1 et 2 passe dans l'oeil en passant dans une canule 4 respective, qui assure la traversée des parois délimitant la cavité intraoculaire, à savoir la sclère et/ou la conjonctive.

La canule 4 est constituée d'une partie tubulaire définissant un passage qui s'étend entre une ouverture 5 proximale destinée à être à l'extérieur du volume intra oculaire et une ouverture distale opposée à l'ouverture 5 et destinée à se trouver dans le volume intra oculaire.

La canule 4 est bloquée en une position dans laquelle elle traverse les deux parois et y reste bloquée par des moyens de blocage qui sont constitués ici par une gorge 6 annulaire creusée dans la surface ou enveloppe extérieure de la canule. La canule des Figures 1 et 2 est représentée plus en détail à la Figure 3. Elle est constituée d'un élément tubulaire ayant un plus grand diamètre extérieur et définit en son sein un canal de passage ayant un diamètre intérieur. La gorge 6 est creusée à la surface extérieure de la canule sensiblement en son milieu. La gorge 6 forme ainsi un rétrécissement dont le diamètre extérieur est inférieur au diamètre extérieur le plus grand de la canule et légèrement supérieur au diamètre intérieur du canal. L'extension en longueur, c'est-à-dire entre les ouvertures 5 proximale et distale le long de la direction longitudinale du canal de la canule, de la rainure 6 correspond sensiblement à l'épaisseur des deux parois formée par la sclère et la conjonctive, de sorte qu'une fois la canule insérée à travers ces deux parois, ces deux parois sont prises en sandwich entre les deux épaulements 10 supérieur et 11 inférieur délimitant la gorge 6 et bloquent ainsi en position la canule 4. Le contact entre la face 12 distale plane de l'épaulement 10 supérieur ou proximal et la paroi de la sclère et/ou de la conjonctive se fait suivant un plan au moins en partie. Le contact entre la face 13 plane proximale de l'épaulement 11 distal ou inférieur et la paroi délimitant l'espace intra oculaire (la sclère et/ou la conjonctive) se fait suivant un plan au moins en partie.

La distance entre les deux faces 12 et 13 planes distale et proximale respectivement des épaulements 10 et 11 proximal et distal est sensiblement égale ou légèrement inférieure à l'épaisseur de la paroi ou des parois délimitant l'espace intra oculaire (la sclère et/ou la conjonctive).

De même aux modes de réalisation des figures 4 ou 5, l'épaulement inférieur ou distal 8 a une face proximale équivalente à la face 13 de l'épaulement 11 du mode de réalisation des figures 1 à 3 et l'épaulement 7 a une face distale équivalent à la face 12 de l'épaulement 11 et les deux épaulements 7 et 8 prennent en sandwich les deux parois délimitant la cavité intra oculaire suivant des contacts plans respectifs, comme dans le mode de réalisation des figures 1 à 3.

Suivant un autre mode de réalisation représenté à la Figure 4, la canule, toujours constituée d'un élément tubulaire formant en son sein un canal, comporte une tête 7 de plus grand diamètre que le diamètre de la partie tubulaire de la canule. D'autre part, une butée 8 annulaire fait saillie à l'extérieur de la canule. Il est ainsi formé entre la tête 7 et la butée 8 annulaire une gorge annulaire, qui a la même fonction que la gorge 6 dans le mode de la réalisation de la Figure 3. En particulier, la même épaisseur peut être prévue pour cette gorge que celle prévue pour la gorge 6 du mode de réalisation de la Figure 3.

On a représenté à la figure un diamètre extérieur de la butée inférieure à celui de la tête. Cependant, tout en restant dans le domaine de l'invention, on peut prévoir des diamètres identiques ou bien à l'inverse un diamètre plus grand pour la butée. Cependant, le fait de prévoir un diamètre plus petit pour la butée présente l'avantage supplémentaire de faciliter la mise en place de la canule.

A la Figure 5, il est représenté encore un autre mode de réalisation correspondant sensiblement à celui de la Figure 4, mais dans lequel, en outre, l'ouverture 9 distale de la canule a une forme en biseau, qui assure une insertion progressive et non traumatique dans l'incision des parois de la sclère et de la conjonctive lorsque la canule est insérée pour la première fois pour être disposée à travers ces parois.

Cette incision peut être réalisée en montant la canule sur un couteau qui incise l'épaisseur sclère+conjonctive, puis au moyen d'une pince le chirurgien maintient la tête de la canule et retire le couteau, la canule étant ainsi mise en place. Il s'agit du système dit « One Step ». Il existe d'autre système, par exemple un système qui consiste à positionner un gabarit qui permet de localiser le pars plana (là où doit être faite l'incision), d'inciser avec un couteau, puis d'insérer la canule, le gabarit aidant à retrouver l'endroit de l'incision Ce système est dit « Two Step » par un outil que l'on introduit dans la canule puis que l'on retire une fois la canule installée.

La canule est fabriquée en un matériau classique dans le domaine, en particulier en métal, tel qu'en acier inoxydable, ou en matière thermoplastiques ou plastiques tel qu'un polyimide.

## Revendications

1. Canule destinée à traverser la conjonctive et la sclère de l'oeil pour permettre le passage d'au moins un outil destiné à avoir une action dans le volume intraoculaire, tel qu'un instrument d'aspiration, un instrument de coupe, par exemple à grande vitesse, du gel vitreux contenu dans le volume intraoculaire, un guide lumineux destiné à transmettre de la lumière pour voir ce qui se passe dans le volume intraoculaire ou analogue, la canule comportant une partie tubulaire définissant un canal de passage pour l'outil qui s'étend entre une ouverture proximale destinée à être à l'extérieur du volume intra oculaire et une ouverture distale destinée à être à l'intérieur du volume intra oculaire, des moyens pour bloquer en position la canule dans une position dans laquelle elle traverse la sclère et la conjonctive, le blocage s'opposant au déplacement de la canule vers l'extérieur et vers l'intérieur du volume intraoculaire, les moyens de blocage ayant la forme d'une gorge annulaire formée à l'extérieur de la canule, de sorte qu'une fois la canule insérée à travers les deux parois de la conjonctive et de la sclère, ces dernières sont prises en sandwich entre deux épaulements (7, 8) annulaires supérieur et inférieur suivant des contacts plans respectifs, la gorge annulaire étant définie entre les deux épaulements (7, 8) annulaires supérieur et inférieur, l'épaulement (8) annulaire inférieur ayant une face supérieure et une face inférieure, et en section transversale longitudinale, la face supérieure de l'épaulement (8) annulaire inférieur et la face inférieure de l'épaulement (7) annulaire supérieur faisant un angle sensiblement perpendiculaire par rapport à la partie tubulaire, la partie tubulaire ayant une partie distale s'étendant à partir de la face inférieure de l'épaulement (8) annulaire inférieur en direction de l'ouverture distale du canal de passage formé dans la partie tubulaire, l'épaulement (8) annulaire inférieur étant inamovible par rapport à la partie tubulaire, **caractérisée en ce que** la partie tubulaire a une section transversale constante à partir de l'épaulement (7) annulaire supérieur en direction de l'ouverture distale.

2. Canule suivant la revendication 1, **caractérisée en ce que** la gorge est formée par deux butées annulaires faisant saillie latéralement de la canule à distance l'une de l'autre, de manière à former entre elles la gorge annulaire.

3. Canule suivant l'une des revendications 1 et 2, **caractérisée en ce que** l'extrémité distale de la canule, c'est-à-dire l'extrémité par laquelle pénètre la canule dans l'espace intraoculaire est de forme biseautée.

4. Ensemble constitué d'au moins une canule suivant l'une des revendications précédentes et d'au moins un outil destiné à avoir une action dans le volume intraoculaire, tel qu'un instrument d'aspiration, un instrument de coupe, par exemple à grande vitesse, du gel vitreux contenu dans le volume intraoculaire, un guide lumineux destiné à transmettre de la lumière pour voir ce qui se passe dans le volume intraoculaire ou analogue, et ayant les dimensions appropriées pour pouvoir passer dans le canal de la canule.

5. Ensemble constitué d'au moins une canule suivant l'une des revendications 1 à 3 et d'au moins un outil destiné à avoir une action dans le volume intraoculaire, tel qu'un instrument d'aspiration, un instrument de coupe, par exemple à grande vitesse, du gel vitreux contenu dans le volume intraoculaire, un guide lumineux destiné à transmettre de la lumière pour voir ce qui se passe dans le volume intraoculaire ou analogue, et passant dans le canal de la canule.

## Patentansprüche

1. Kanüle, die dazu bestimmt ist, durch die Bindehaut und die Sklera des Auges zu gelangen, um den Durchgang mindestens eines Werkzeugs zu ermöglichen, das dazu bestimmt ist, in den intraokularen Volumen zu wirken, wie beispielsweise ein Aspirationsinstrument, ein Schneidinstrument, beispielsweise mit hoher Geschwindigkeit, zum Schneiden des im intraokularen Volumen enthaltenen Glasgels, einen Lichtleiter, der dazu bestimmt ist, Licht zu übertragen, um zu sehen, was im intraokularen Volumen oder dergleichen geschieht, wobei die Kanüle einen rohrförmigen Teil aufweist, der einen Durchgangskanal für das Werkzeug definiert, die sich zwischen einer proximalen Öffnung, die dazu bestimmt ist, außerhalb des intraokularen Volumens zu liegen, und einer distalen Öffnung, die dazu bestimmt ist, innerhalb des intraokularen Volumens zu liegen, erstreckt, Mittel zum Blockieren einer Position der Kanüle in einer Position, in der sie durch die Sklera und die Bindehaut verläuft, wobei die Blockierung verhindert, dass sich die Kanüle nach außen und nach innen des intraokularen Volumens bewegt, wobei die Blockierungsmittel in Form einer Ringnut ausgebildet sind, die an der Außenseite der Kanüle ausgebildet ist, so dass, nachdem die Kanüle durch die beiden Wände der Bindehaut und der Sklera eingeführt wurde, diese zwischen zwei oberen und unteren ringförmigen Schultern (7,8), die jeweils in planarem Kontakt stehen, eingeklemmt sind, wobei die Ringnut zwischen den beiden oberen und unteren ringförmigen Schultern (7,8) definiert ist, wobei die untere ringförmige Schulter (8) eine obere Fläche und eine untere Fläche aufweist, und im Längsschnitt die obere Fläche der unteren ringförmigen Schulter (8) und die untere Fläche der oberen ringförmigen Schulter (7) einen Winkel im Wesentlichen senkrecht zu dem Rohrteil bilden, wobei der rohrförmige Teil einen distalen Teil aufweist, der sich von der Unterseite der unteren ringförmigen Schulter (8) in Richtung der distalen Öffnung des im rohrförmigen Teils gebildeten Durchgangskanals erstreckt, wobei die untere ringförmige Schulter (8) in Bezug auf den rohrförmigen Teil unbeweglich ist, **dadurch gekennzeichnet, dass** der rohrförmige Teil einen Querschnitt aufweist, der von der oberen ringförmigen Schulter (7) in Richtung der distalen Öffnung konstant ist.

2. Kanüle nach Anspruch 1, **dadurch gekennzeichnet, dass** die Nut durch zwei ringförmige Anschläge gebildet ist, die seitlich aus der Kanüle in einem Abstand voneinander herausragen, um die Ringnut zwischen einander zu bilden.

3. Kanüle nach einem der Ansprüche 1 und 2, **dadurch gekennzeichnet, dass** das distale Ende der Kanüle, d.h. das Ende, mit dem die Kanüle in den intraokularen Raum gelangt, eine abgeschrägte Form aufweist.

4. Anordnung, bestehend aus mindestens einer Kanüle nach einem der vorstehenden Ansprüchen und mindestens einem Werkzeug, das dazu bestimmt ist, in dem intraokularen Volumen zu wirken, wie beispielsweise ein Aspirationsinstrument, ein Schneideinstrument, beispielsweise ein Hochgeschwindigkeits-Schneideinstrument, zum Schneiden des im intraokularen Volumen enthaltenen glasartigen Gels, einem Lichtleiter, der dazu bestimmt ist, Licht zu übertragen, um zu sehen, was im intraokularen Volumen oder dergleichen geschieht, und der geeignete Abmessungen aufweist, um in den Kanal der Kanüle eintreten zu können.

5. Anordnung, bestehend aus mindestens einer Kanüle nach einem der Ansprüche 1 bis 3 und mindestens einem Werkzeug, das dazu bestimmt ist, im intraokularen Volumen zu wirken, wie beispielsweise ein Aspirationsinstrument, ein Schneideinstrument, beispielsweise mit hoher Geschwindigkeit, zum Schneiden des im intraokularen Volumen enthaltenen glasartigen Gels, einem Lichtleiter, der dazu bestimmt ist, Licht zu übertragen, um zu sehen, was im intraokularen Volumen oder dergleichen geschieht, und in den Kanal der Kanüle hineintritt.

## Claims

1. Cannula intended to traverse the conjunctiva and the sclera of the eye in order to enable the passage of at least one tool intended to have an action in the intraocular volume, such as a suction instrument, an instrument for cutting - for example at high speed - the vitreous gel contained in the intraocular volume, a light-guide intended to transmit light in order to see what is happening in the intraocular volume or similar, the cannula including a tubular part defining a passage channel for the tool, which extends between a proximal opening intended to be outside the intraocular volume and a distal opening intended to be inside the intraocular volume, means being provided for locking the cannula in position in a position in which it traverses the sclera and the conjunctiva, the locking opposing the displacement of the cannula towards the outside and towards the inside of the intraocular volume, the locking means having the form of an annular groove formed on the outside of the cannula so that, once the cannula has been inserted through the two walls of the conjunctiva and of the sclera, these latter are sandwiched between two upper and lower annular shoulders (7, 8) along respective plane contacts, said annular groove being defined between the lower and upper annular shoulders (7, 8), said lower annular shoulder (8) having an upper face and a lower face, and in longitudinal cross section, the upper face of lower annular shoulder (8) and the lower face of upper annular shoulder (7) making a substantially perpendicular angle relative to the tubular part, said tubular part having a distal part extending from said lower face of said lower annular shoulder (8) towards said distal opening of the passage channel formed inside said tubular part, said lower annular shoulder (8) being fixed relative to said tubular part, **characterised in that** said tubular part has a cross section which is constant from said upper annular shoulder (7) towards said distal opening.

2. Cannula according to Claim 1, **characterised in that** the groove is formed by two annular stops projecting laterally from the cannula and spaced from one another so as to form the annular groove therebetween.

3. Cannula according to one of Claims 1 and 2, **characterised in that** the distal end of the cannula - that is to say the end through which the cannula penetrates into the intraocular space - is of bevelled shape.

4. Assembly constituted by at least one cannula according to one of the preceding claims and by at least one tool intended to have an action in the intraocular volume, such as a suction instrument, an instrument for cutting - for example at high speed - the vitreous gel contained in the intraocular volume, a light-guide intended to transmit light in order to see what is happening in the intraocular volume or similar, and having appropriate dimensions in order to be able to pass into the channel of the cannula.

5. Assembly constituted by at least one cannula according to one of Claims 1 to 3 and by at least one tool intended to have an action in the intraocular volume, such as a suction instrument, an instrument for cutting - for example at high speed - the vitreous gel contained in the intraocular volume, a light-guide intended to transmit light in order to see what is happening in the intraocular volume or similar, and passing into the channel of the cannula.
